# EUROPÄISCHE PATENTSCHRIFT

(11) **EP 2 248 541 B1**
(45) Veröffentlichungstag und Bekanntmachung des Hinweises auf die Patenterteilung: **31.10.2018**
(21) Anmeldenummer: 10156621.4
(22) Anmeldetag: 16.03.2010
(51) Int. Cl.: A61L 29/08, A61L 29/16

(54) **MEDIKAMENTENBESCHICHTETER BALLONKATHETER UND VERFAHREN ZUR HERSTELLUNG DESSELBEN**
MEDICATION-COATED BALLOON CATHETER AND METHOD FOR MANUFACTURING THE SAME
CATHÉTER À BALLONNET REVÊTU DE MÉDICAMENT ET SON PROCÉDÉ DE FABRICATION

(30) Priorität: 07.05.2009 DE 102009002893
(43) Veröffentlichungstag der Anmeldung: 10.11.2010
(73) Patentinhaber: BIOTRONIK AG, 8180 Bülach (CH)
(72) Erfinder: Tittelbach, Michael, 90429 Nürnberg (DE); Moehl, Raimund, 8127 Forch (CH); Schwitzer, Alwin, 8180 Bülach (CH); Wesselmann, Matthias, 8912 Glattfelden (CH); Quint, Bodo, 8154 Oberglatt (CH); Bachmann, Patrice, 8400 Winterthur (CH)
(74) Vertreter: Keck, Hans-Georg

(56) Entgegenhaltungen:
- EP-A2- 0 357 369
- WO-A1-95/05866
- US-A1- 2006 280 858
- US-A1- 2007 202 147
- US-A1- 2007 280 988
- Suzanna Pereira Nunes ET AL: "Chapter I-3. Membrane Preparation", Membrane Technology: in the Chemical Industry, 1. Januar 2001 (2001-01-01), Seiten 6-11, XP055137677, Weinheim, Germany DOI: 10.1002/3527600388.ch3 ISBN: 978-3-52-760038-0 Gefunden im Internet: URL:http://onlinelibrary.wiley.com/store/1 0.1002/3527600388.ch3/asset/ch3.pdf?v=1&t= hzl05k4q&s=92d03e0c38c157f0dbbb87e791c5e0f 4d4222881 [gefunden am 2014-09-02]

## Beschreibung

Die Erfindung betrifft einen medikamentenbeschichteten Ballonkatheter sowie ein Verfahren zur Herstellung desselben.

Der Einsatz von Ballonkathetern ist bei unterschiedlichsten Indikationen in vielen Bereichen der Medizintechnik ein bevorzugtes therapeutisches Verfahren. So wird beispielsweise in der Angiologie und Kardiologie eine Aufweitung verengter Blutgefäße mittels Ballondilatation unter gleichzeitiger Freisetzung restenosehemmender Medikamente vorgeschlagen. Nach einer Variante werden dazu die Medikamente direkt auf den zu dilatierenden Ballon aufgebracht. Es hat sich jedoch gezeigt, dass in der Praxis bis zu 80% des anhaftenden Medikaments nicht an der gewünschten Stelle des Gefäßes appliziert wird, sondern von der im Lumen befindlichen Körperflüssigkeit vorher herausgelöst und fortgetragen wird. Dies erhöht die Gefahr unerwünschter systemischer Nebenwirkungen der Medikamente.

Als Gegenmaßnahme ist beispielsweise vorgeschlagen worden, die Expansion des Ballonkatheters über eine hantelförmige Zwischenstufe verlaufen zu lassen, in der die beiden Enden des Ballonkatheters den mit Medikamenten beschichteten Zwischenbereich gegenüber dem Lumen des Gefäßes abschirmen. Anschließend wir der Ballon vollständig expandiert. Ein solcher, als Zwischenstufe hantelförmiger Ballonkatheter ist jedoch in seiner Herstellung und Handhabung komplex und damit fehleranfällig. Zudem ist bei unregelmäßigen Gefäßgeometrien dennoch ein Ausspülen des Medikaments nicht zu verhindern. WO 95/05866 beschreibt einen Ballon mit einer Beschichtung. US 2007/0202147 A1 offenbart einen Stent mit einer Beschichtung. US 2007/0280988 A1 beschreibt ebenfalls Stents mit Beschichtung.

Ein anderer Ansatz wäre der Auftrag einer das Medikament aufnehmenden oder abdeckenden Beschichtung. Die Herstellung einer solchen Beschichtung ist jedoch aufwendig und derartig beschichtete Katheter können in der Regel nicht lange gelagert werden. Das Beschichtungsmaterial muss biokompatibel sein und die Eigenschaften von Medikament und Beschichtungssystem müssen in jedem Einzelfall aufeinander abgestimmt werden. In der Praxis ist damit die Realisierung eines solchen medikamentenbeschichteten Ballonkatheters sehr aufwendig und bietet dennoch nicht das gewünschte Maß an Sicherheit für die lokale Administration des Medikaments.

Der Erfindung liegt daher die Aufgabe zugrunde, einen medikamentenbeschichteten Katheter bereitzustellen, der eines oder mehrere der angesprochenen Probleme löst oder zumindest mindert.

Ein erster Aspekt der Erfindung liegt in der Bereitstellung eines medikamentenbeschichteten Ballonkatheters, dessen Ballon
(i) eine Basismembran und
(ii) eine auf einer Außenseite der Basismembran asymmetrische Polymermembran, in die zumindest ein pharmazeutischer Wirkstoff eingebracht ist,
   umfasst.

Der erfindungsgemäße Ballonkatheter besitzt demnach eine mikroporöse asymmetrische Polymermembran auf der Außenseite der Ballonwandung. Diese Polymermembran weist eine Vielzahl von Poren und Mikrofurchen auf, in die das zu applizierende Medikament in gelöster Form oder Reinform eingebettet ist. Asymmetrische Polymermembranen zeichnen sich in ihrer Morphologie dadurch aus, dass sie an ihrer Außenseite eine höhere Dichte aufweisen als an ihrer Basis, d.h. der Basismembran zugewandten Seite. Eine Zugänglichkeit der Kavitäten der Polymermembran ist im nicht-expandierten Zustand des Ballons so gering, dass eine Ausspülung des Medikaments durch die Körperflüssigkeit stark gemindert oder gar verhindert wird. Erst im expandierten Zustand sind die Poren und Mikrofurchen an der Oberseite der Polymermembran derart aufgeweitet, dass eine Freisetzung des inkorporierten Wirkstoffs problemlos erfolgen kann.

Asymmetrische Polymermembranen weisen demnach eine dünne Deckschicht mit brauchbarer mechanischer Stabilität auf, die eine darunter liegende poröse Struktur schützt. Der Begriff "asymmetrische Membran" fasst eben diese Morphologie begrifflich zusammen und wird ebenso in der Literatur verwendet (siehe zum Beispiel Membrane Technology in the Chemical Industry, 2001 Wiley-VCH Verlag GmbH, Kapitel 3, Seiten 6 - 11). Die Struktur asymmetrischer Polymermembranen ist geprägt durch ihr Herstellungsverfahren. Daher richtet sich ein weiterer Aspekt der Erfindung auf die Herstellung eines solchen medikamentenbeschichteten Ballonkatheters. Das Verfahren umfasst die Schritte
a) Bereitstellen eines Ballonrohlings mit einer Basismembran;
b) Benetzen der Basismembran mit einer homogenen Polymerlösung aus einem Lösungsmittel und einem Polymer;
c) Induzierung einer Phasenseparation des Polymers aus der Polymerlösung zur Abscheidung einer asymmetrischen Polymermembran durch eine der Maßnahmen
   (i) Temperaturwechsel,
   (ii) Eintauchen des benetzten Ballonrohlings in ein Bad aus einer mit dem Lösungsmittel der Polymerlösung mischbaren, aber das Polymer schlechter oder nicht lösenden Flüssigkeit (Nassverfahren) oder
   (iii) Aussetzen des benetzten Ballonrohlings einer Atmosphäre, die eine mit dem Lösungsmittel der Polymerlösung mischbare, aber das Polymer schlechter oder nicht lösende gasförmige Komponente enthält (Trockenverfahren).

Das Verfahren geht demnach nach Schritt a) zunächst von einem Ballonkatheter mit Ballon aus, dessen äußere Membranwandung zu beschichteten ist. Für die Zwecke der Erfindung ist es dabei unerheblich, ob es sich um einen Monolumen-, Mehrlumen- oder Multilayer-Katheter handelt. In jedem Fall wird die Außenseite des Ballons, im Weiteren als Basismembran bezeichnet, durch Abscheiden der asymmetrischen Polymermembran modifiziert.

Die Basismembran besteht vorzugsweise zumindest an ihrer Außenseite aus einem für die Zwecke üblicherweise verwendeten polymeren Material, insbesondere ist das polymere Material der Basismembran ausgewählt aus der Gruppe umfassend Polyurethan, Polyether-Polyurethan, Polyethylenterephthalat, Polybutylenterephthalat, Polyamid sowie Copolymere und Blends derselben. Polyamide sind besonders bevorzugt, da sie eine besonders hohe Festigkeit aufweisen.

Typische Ballonmaterialien sind meist semikristalline Thermoplaste, wobei im PTCA / PTA Bereich primär Polyamide, Polyethylenterephthalat (PET) oder Polybutylenterephthalat (PBT) sowie deren Copolymere und Blends eingesetzt werden. Polyurethane setzen sich als alternative Materialien zunehmend für dehnbare und anpassungsfähige Ballonapplikationen durch. Typische Okklusionsballone bestehen häufig aus Latex.

Weiterhin wird eine homogene Polymerlösung aus einem Lösungsmittel mit einem für den Aufbau der asymmetrischen Polymermembran geeigneten Polymer bereitgestellt. Besonders bevorzugte Polymere sind Polyurethan oder Polyether-Polyurethan. Es können jedoch auch andere, in einem Lösungsmittel homogen lösbare Polymere verwendet werden, zum Beispiel aromatische Polyimide, Polyethersulfone, Polypropylen, Cellulose und Cellulosederivate.

Das Lösungsmittel ist derart auszuwählen, dass eine für das Verfahren hinreichend hohe Konzentration des Polymers ermöglicht. Weiterhin haben die Eigenschaften des Lösungsmittels erheblichen Einfluss auf die Phaseninversion. Geeignete Lösungsmittel umfassen insbesondere Dimethylformamid (DMF) und Tetrahydrofuran (THF). Letzteres ist besondert bevorzugt, da es sich aufgrund seines relativ niedrigen Siedepunktes und seiner guten Wasserlöslichkeit wieder gut aus dem Produkt entfernen lässt. In den genannten Lösungsmitteln haben insbesondere Polyurethane und Polyether-Polyurethane eine besonders hohe Löslichkeit.

Die homogene Polymerlösung wird im Schritt b) auf den zu beschichteten Bereich des Ballonkatheters aufgebracht, sei es beispielsweise durch Eintauchen in die Lösung oder Aufsprühen derselben. Die Benetzung kann insbesondere im expandierten Zustand des Ballonrohlings erfolgen, so dass sich die oberflächennahen Bereiche der Polymermembran bei Deflation weiter verdichten und eine Zugänglichkeit der inneren Struktur von Außen erschweren.

Eingeleitet wird der Prozess der Phaseninversion der zunächst homogenen Polymerlösung durch Temperaturwechsel (Alternative (i) Schritt c)), Eintauchen des benetzten Ballonrohlings in ein Bad aus einer mit dem Lösungsmittel der Polymerlösung mischbaren, aber das Polymer schlecht oder nicht lösbaren Flüssigkeit (Nassverfahren; Alternative (ii) Schritt c)) oder Aussetzen des vernetzten Ballonrohlings einer Atmosphäre, die eine mit dem Lösungsmittel mischbare, aber das Polymer schlecht oder nicht lösende gasförmige Komponente enthält (Trockenverfahren; Alternative (iii) Schritt c)).

In der thermischen Prozessführung agiert üblicherweise eine niedermolekulare Verbindung als Lösungsmittel bei hohen Temperaturen, vermag das Polymer aber nur unzureichend bei niedrigeren Temperaturen zu lösen. Eine derartige Prozessführung bietet sich immer dann an, wenn das abzuscheidende Polymer schlechte Lösungseigenschaften besitzt, wie z.B. Polypropylen.

Die isotherme Phasenseparation, insbesondere nach dem Trockenverfahren, ist jedoch vorliegend bevorzugt. Bei dieser Verfahrensführung wird die Polymerlösung einer flüssigen oder gasförmigen Komponente ausgesetzt, die sich von der Außenseite des flüssigen Polymerfilms ausgehend allmählich in der polymeren Lösung ausbreitet. Diese Komponente kann das Polymer jedoch nicht oder nur schlechter lösen, so dass das Polymer durch Phasenseparation ausfällt. Besonders einfach realisieren lässt sich ein solches System beispielsweise beim Einsatz einer THF-Polymerlösung: Hier kann Wasser oder ein Wasser/Alkohol-Gemisch als Nichtlösungsmittel für das Polymer Verwendung finden.

Die Morphologie der sich im Zuge des Verfahrens bildenden asymmetrischen Polymermembran lässt sich durch die gewählten Verfahrensparameter beeinflussen. Die dem Prozess zugrunde liegenden Mechanismen sind komplex und bisher nicht abschließend geklärt. Für die Praxis sind jedoch Parameter bekannt, die zu Strukturen mit überwiegend schaumartig, mikroporösen Charakter oder fingerartigen Kavitäten / Mikrofurchen führen. Die schaumartige Struktur bildet sich bevorzugt, wenn die Polymerkonzentration in der Polymerlösung ansteigt, die Viskosität der Polymerlösung z.B. durch Zusatz eines Vernetzers erhöht wird oder Gemische aus Lösungsmittel und Nichtlösungsmitteln verwendet werden. Für die vorliegenden Zwecke ist eine Struktur bevorzugt, die eine Mischung aus beiden Strukturtypen darstellt.

Der pharmazeutische Wirkstoff kann im einfachsten Falle bereits in der Polymerlösung enthalten sein. Hierdurch wird der Herstellungsprozess stark vereinfacht. Es ist jedoch auch denkbar, nach der Erzeugung der asymmetrischen Polymermembran auf die getrocknete und gereinigte Polymermembran eine Lösung eines pharmazeutischen Wirkstoffs oder den reinen Wirkstoff aufzutragen. Diese Auftragung sollte insbesondere im expandierten Zustand des Ballons erfolgen, um eine Einbettung des Materials in das innere Lumen der Polymerschicht zu erleichtern. Der Wirkstoff beziehungsweise die Wirkstofflösung besitzt idealerweise nur minimale Wechselwirkungen mit der Polymermembran, um die Freisetzung zu erleichtern. Dies kann beispielsweise durch Zusatz geeigneter Additive zur Polymerlösung erreicht werden.

Die Erfindung wird nachfolgend anhand eines Ausführungsbeispiels und den dazugehörigen Zeichnungen näher erläutert. Es zeigen:
- Figur 1: eine Abbildung der Ballonmembran eines nach dem erfindungsgemäßen Verfahren modifizierten Ballonkatheters; und
- Figur 2: einen vergrößerten Ausschnitt der Oberfläche der Ballonmembran aus Figur 1

### Herstellung eines beschichteten Ballonkatheters

Ein Ballonkatheter mit einer Ballonmembran aus Polyamid wurde distal mit einem Silikonschlauch verschlossen, mit geringem Druck (3 - 5 bar) inflatiert und in eine Lösung getaucht. Der Katheter wurde in einer kontinuierlichen und langsamen Bewegung aus der Tauchlösung herausgezogen, wobei zu beachten ist, dass die Geschwindigkeit des Herausziehens und die Viskosität der Polymerlösung einen Einfluss auf die applizierte Schichtdicke der zu erzeugenden Polymermembran hat.

Die Koagulation des Polymers aus der Polymerlösung erfolgt durch Einbringen des mit der Polymerlösung beschichteten Ballonkatheters in eine Konditionierkammer mit einer Atmosphäre aus Isopropanol/Wasser (hergestellt durch Erhitzen einer 50/50 Isopropanol/Wasser-Mischung bei 70°C).

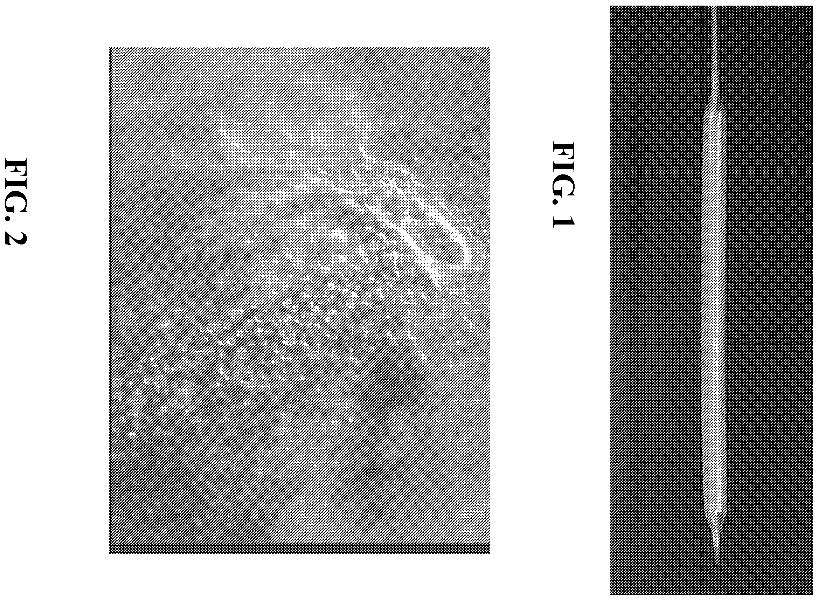

Nach 10 Minuten wurde der Ballon der Konditionierkammer entnommen, mit destilliertem Wasser mehrmals gespült und getrocknet. Die Figuren 1 und 2 illustrieren die morphologischen Veränderungen auf der Oberfläche des Ballonkatheters.

Der konische Bereich des Ballons, welcher für die Beschichtung interessant ist, wird proximal durch die Tiefe des Eintauchens in die Polymerlösung und die distale nachträgliche Entfernung der Polymerbeschichtung mit Hilfe eines Lösungsmittels definiert.

Der Ballonkatheter kann nun im inflatierten Zustand mit einer Wirkstofflösung benetzt werden, die in die erzeugten Kavitäten der Polymermembran eingebettet wird.

## Patentansprüche

1. Medikamentenbeschichteter Ballonkatheter mit einem Ballon umfassend:
(i) eine Basismembran und
(ii) eine auf einer Außenseite der Basismembran aufgebrachte asymmetrische Polymermembran, in die zumindest ein pharmazeutischer Wirkstoff eingebettet ist.

2. Katheter nach Anspruch 1, bei dem die Basismembran zumindest an ihrer der Polymermembran zugewandten Seite aus einem polymeren Material besteht.

3. Katheter nach Anspruch 2, bei dem das polymere Material der Basismembran ausgewählt ist aus der Gruppe umfassend Polyurethan, Polyether-Polyurethan, Polyethylenterephthalat, Polybutylenterephthalat, Polyamid sowie Copolymere und Blends derselben.

4. Katheter nach Anspruch 3, bei dem das polymere Material der Basismembran Polyamid ist.

5. Katheter nach einem der vorhergehenden Ansprüche, bei dem die Polymermembran aus Polyurethan oder Polyether-Polyurethan besteht.

6. Katheter nach einem der vorhergehenden Ansprüche, bei dem zumindest ein pharmazeutischer Wirkstoff die Polymermembran eingebettet ist.

7. Verfahren zur Herstellung des medikamentenbeschichteten Ballonkatheters nach Anspruch 1, umfassend die Schritte:
a) Bereitstellen eines Ballonrohlings mit einer Basismembran;
b) Benetzen der Basismembran mit einer homogenen Polymerlösung aus einem Lösungsmittel und einem Polymer;
c) Induzierung einer Phasenseparation des Polymers aus der Polymerlösung zur Abscheidung einer asymmetrischen Polymermembran auf der Basismembran durch eine der Maßnahmen
(i) Temperaturwechsel,
(ii) Eintauchen des benetzten Ballonrohlings in ein Bad aus einer mit dem Lösungsmittel der Polymerlösung mischbaren, aber das Polymer schlechter oder nicht lösenden Flüssigkeit (Nassverfahren) oder
(iii) Aussetzen des benetzten Ballonrohlings einer Atmosphäre, die eine mit dem Lösungsmittel der Polymerlösung mischbare, aber das Polymer schlechter oder nicht lösende gasförmige Komponente enthält (Trockenverfahren).

8. Verfahren nach Anspruch 7, bei dem Schritt c) in einem expandierten Zustand des Ballonrohlings erfolgt.

9. Verfahren nach Anspruch 8, bei dem die Polymerlösung einen pharmazeutischen Wirkstoff enthält.

10. Verfahren nach Anspruch 7 oder 8, bei dem der Ballon nach der Phasenseparation getrocknet und anschließend ein pharmazeutischer Wirkstoff durch Auftragen einer Wirkstofflösung oder eines reinen Wirkstoffs auf die Polymermembran im expandierten Zustand des Ballonrohlings eingebettet wird.

11. Verfahren nach einem der Ansprüche 7 bis 10, bei dem die Basismembran des Ballons zumindest an ihrer der Polymermembran zugewandten Seite aus Polyamid besteht und die Polymerlösung eine Lösung von Polyurethan oder Polyether-Polyurethan in Tetrahydrofuran (THF) oder Dimethylformamid (DMF) ist.

## Claims

1. A drug-coated balloon catheter having a balloon, comprising:
(i) a main membrane, and
(ii) an asymmetrical polymer membrane which is applied to an outside of the main membrane and into which at least one pharmaceutical active ingredient is embedded.

2. The catheter according to claim 1, wherein the main membrane comprises a polymer material at least on the side thereof facing the polymer membrane.

3. The catheter according to claim 2, wherein the polymer material of the main membrane is selected from the group consisting of polyurethane, polyether-polyurethane, polyethylene terephthalate, polybutylene terephthalate, polyamide, and copolymers and blends thereof.

4. The catheter according to claim 3, wherein the polymer material of the main membrane is polyamide.

5. A catheter according to any one of the preceding claims, wherein the polymer membrane comprises polyurethane or polyether-polyurethane.

6. A catheter according to any one of the preceding claims, wherein at least one pharmaceutical active ingredient is embedded into the polymer membrane.

7. A method for producing a drug-coated balloon catheter according to claim 1, comprising the following steps:
a) providing a balloon blank having a main membrane;
b) wetting the main membrane with a homogeneous polymer solution comprising a solvent and a polymer;
c) inducing a phase separation of the polymer from the polymer solution for deposition of an asymmetrical polymer membrane on the main membrane by one of the following measures:
(i) Temperature change,
(ii) immersing the wetted balloon blank in a bath of a liquid which can be mixed with the solvent of the polymer solution, but which does not dissolve, or hardly dissolves, the polymer (wet process), or
(iii) exposing the wetted balloon blank to an atmosphere which comprises a gaseous constituent which can be mixed with the solvent of the polymer solution, but does not dissolve, or hardly dissolves, the polymer (dry method).

8. The method according to claim 7, wherein step c) is carried out in an expanded state of the balloon blank.

9. The method according to claim 8, wherein the polymer solution comprises a pharmaceutical active ingredient.

10. The method according to claim 7 or 8, wherein the balloon is dried after the phase separation, and subsequently a pharmaceutical active ingredient is embedded by applying an active ingredient solution, or a pure active ingredient, onto the polymer membrane in the expanded state of the balloon blank.

11. The method according to any one of claims 7 to 10, wherein the main membrane of the balloon comprises polyamide at least on the side thereof facing the polymer membrane, and the polymer solution is a solution of polyurethane or polyether-polyurethane in tetrahydrofurane (THF) or dimethylformamide (DMF).

## Revendications

1. Cathéter à ballon revêtu de médicaments doté d'un ballon, comprenant :
(i) une membrane de base et
(ii) une membrane polymère asymétrique rapportée sur une face extérieure de la membrane de base dans laquelle est incorporée au moins une matière active pharmaceutique.

2. Cathéter selon la revendication 1, chez lequel la membrane de base est constituée d'un matériau polymère au moins sur sa face orientée vers la membrane polymère.

3. Cathéter selon la revendication 2, chez lequel le matériau polymère est choisi dans le groupe comprenant le polyuréthane, le polyéther-polyuréthane, le polyéthylène téréphtalate, le polyamide ainsi que des copolymères et mélanges de ceux-ci.

4. Cathéter selon la revendication 3, chez lequel le matériau polymère de la membrane de base est du polyamide.

5. Cathéter selon l'une des revendications précédentes, chez lequel la membrane polymère est constituée de polyuréthane ou de polyéther-polyuréthane.

6. Cathéter selon l'une des revendications précédentes, chez lequel au moins une matière active pharmaceutique est incorporée dans la membrane polymère.

7. Procédé de fabrication du cathéter à ballon revêtu de médicaments selon la revendication 1, comprenant les étapes :
a) de mise à disposition d'une ébauche de ballon avec une membrane de base ;
b) de mouillage de la membrane de base avec une solution de polymère homogène à base d'un solvant et d'un polymère ;
c) d'induction d'une séparation de phases du polymère à partir de la solution de polymère par précipitation d'une membrane de polymère asymétrique sur la membrane de base par l'une des mesures
(i) de changement de température,
(ii) d'immersion de l'ébauche de ballon mouillée dans un bain à base d'un liquide miscible avec le solvant de la solution de polymère, mais dissolvant plus difficilement ou ne dissolvant pas le polymère (procédé par voie humide), ou
(iii) de soumission de l'ébauche de ballon mouillée à une atmosphère qui contient une composante sous forme gazeuse miscible avec le solvant de la solution de polymère, mais dissolvant plus difficilement ou ne dissolvant pas le polymère (procédé par voie sèche).

8. Procédé selon la revendication 7, chez lequel l'étape c) a lieu dans un état expansé de l'ébauche de ballon.

9. Procédé selon la revendication 8, chez lequel la solution de polymère contient une matière active pharmaceutique.

10. Procédé selon la revendication 7 ou 8, chez lequel le ballon est séché après la séparation des phases et ensuite une matière active pharmaceutique est incorporée par application d'une solution de matière active ou d'une matière active pure sur la membrane de polymère dans l'état expansé de l'ébauche de ballon.

11. Procédé selon l'une des revendications 7 à 10, chez lequel la membrane de base du ballon est constituée de polyamide au moins sur sa face orientée vers la membrane de polymère, et la solution de polymère est une solution de polyuréthane ou de polyéther-polyuréthane dans le tétrahydrofuranne (THF) ou le diméthylformamide (DMF).
